# EUROPEAN PATENT APPLICATION

(11) **EP 1 844 738 A1**
(43) Date of publication of application: **17.10.2007**
(21) Application number: 07007338.2
(22) Date of filing: 10.04.2007
(51) Int. Cl.: A61F 2/84

(54) **A mechanism to ensure placement of ostial renal stents**

(30) Priority: 10.04.2006 US 279206
(71) Applicant: Medtronic Vascular, Inc., Santa Rosa, California 95403 (US)
(72) Inventor: Hopkins, Darren, Windsor, CA 95492 (US); Chu, Jack, Santa Rosa, CA 95409 (US); Morris, Jonathan, Keller, TX 76248 (US); Machek, James, Santa Rosa, CA 95404 (US); Ganesan, Prema, Oakland, CA 94609 (US); Greenan, Trevor, Santa Rosa, CA 95405 (US); Rust, Matthew, Santa Rosa, CA 95405 (US); Moriarty, James, Georgetown Massachusetts 01833 (US)
(74) Representative: Zimmermann, Gerd Heinrich

(57) **Abstract**

A delivery system (100) comprises a stent dilation balloon (118) at a distal end (114) of said delivery system (100); a stent (102) on said stent dilation balloon (118); and a basket assembly (116) proximally adjacent to said stent dilation balloon (118), said basket assembly (116) comprising at least one super-elastic self-expanding basket spline (130).

## Description

### BACKGROUND OF THE INVENTION

### FIELD OF THE INVENTION

The present invention relates generally to medical devices and methods. More particularly, the present invention relates to a device for the placement of a structure in a human body.

### DESCRIPTION OF RELATED ART

The ostium of a vessel is an opening, aperture, or orifice located at the point of origin of the vessel. Typically, a vessel branches off from a larger parent vessel. For example, the aorta gives rise to the coronary arteries; the opening at the origin of each coronary artery as it branches from the aorta is referred to as an ostium.

An ostial lesion is a lesion, e.g., artherosclerotic plaque, located at the ostium of a vessel. In the field of interventional cardiology, ostial lesions are stented to maintain patency of the vessel.

To effectively stent an ostial lesion, the stent must be accurately placed to cover the ostial lesion without significantly protruding into the parent vessel. To facilitate the accurate stent placement, radiopaque contrast liquid is often injected into the patient. Although effective in locating and placing the stent, the contrast is cytotoxic and can contribute to complications with the patient such as renal failure.

### SUMMARY OF THE INVENTION

In view of the above, a delivery system according to claim 1 and 36 is provided. Further aspects, features, details, and advantages of the present invention are apparent from the dependent claims, the specification, and the accompanying drawings.

In accordance with one embodiment, a delivery system includes a stent dilation balloon at a distal end of the delivery system; a stent on the stent dilation balloon; and a basket assembly proximally adjacent to the stent dilation balloon, the basket assembly including at least one super-elastic self-expanding basket spline.

In accordance with an example, a method of using the delivery system includes advancing the stent to be located within an ostium of a vessel; advancing a basket actuation button of a handle to deploy a basket of the basket assembly; moving the basket into engagement with a parent vessel, the basket having a larger diameter than a diameter of the ostium; deploying the stent within an ostial lesion of the vessel; and contracting the basket.

The physician using the delivery system feels the engagement of the basket against the wall of the parent vessel thus confirming the correct placement of the stent. The stent is positioned accurately using the delivery system without the use of contrast in one example.

### BRIEF DESCRIPTION OF THE DRAWINGS

FIG. 1 is a perspective view of a basket delivery system for placement of a stent in an ostial lesion in accordance with one embodiment of the present invention;

FIG. 2 is an enlarged perspective view of the region II of the basket delivery system of FIG. 1;

FIG. 3 is an enlarged cross-sectional view of the region II of the basket delivery system of FIG. 1;

FIG. 4 is an enlarged cross-sectional view of the basket delivery system along the line IV-IV of FIG. 3;

FIG. 5 is an enlarged perspective view of the region II of the basket delivery system of FIG. 1 with a basket assembly in its deploying state;

FIG. 6 is an enlarged cross-sectional view of a region of a basket delivery system in accordance with another embodiment;

FIG. 7 is a cross-sectional view of the basket delivery system along the line VII-VII of FIG. 6;

FIG. 8 is a sectional schematic perspective view of the advancement of the basket delivery system of FIGS. 1-5 to an ostial lesion of an artery in accordance with one embodiment of the present invention;

FIG. 9 is a sectional schematic perspective view of the further advancement of the basket delivery system of FIG. 8 to the ostial lesion of the artery;

FIG. 10 is a schematic perspective view of a basket delivery system in accordance with another embodiment;

FIG. 11 is a schematic perspective view of a stent delivery sheath of the basket delivery system of FIG. 10;

FIG. 12 is a perspective view of the basket delivery system of FIG. 10 having a basket deployed;

FIG. 13 is a perspective view of the basket delivery system of FIG. 12 during deployment of a self-expanding stent;

FIG. 14 is an enlarged cross-sectional view of a region of a basket delivery system during advancement to an ostial lesion of an artery in accordance with another embodiment;

FIG. 15 is an enlarged cross-sectional view of the region of the basket delivery system during advancement to the ostial lesion of the artery of FIG. 14 with a basket assembly in its deploying state;

FIG. 16 is an enlarged cross-sectional view of a region of a basket delivery system in accordance with another embodiment;

FIG. 17 is an enlarged cross-sectional view of the region of the basket delivery system of FIG. 16 with a basket assembly in its deploying state;

FIGS. 18, 19, 20 are sectional schematic perspective views of the advancement and deployment of the basket delivery system of FIGS. 16, 17; and

FIGS. 21, 22, 23, 24 and 25 are perspective views of regions of basket delivery systems having baskets in accordance with various other embodiments.

Common reference numerals are used throughout the drawings and detailed description to indicate like elements.

### DETAILED DESCRIPTION

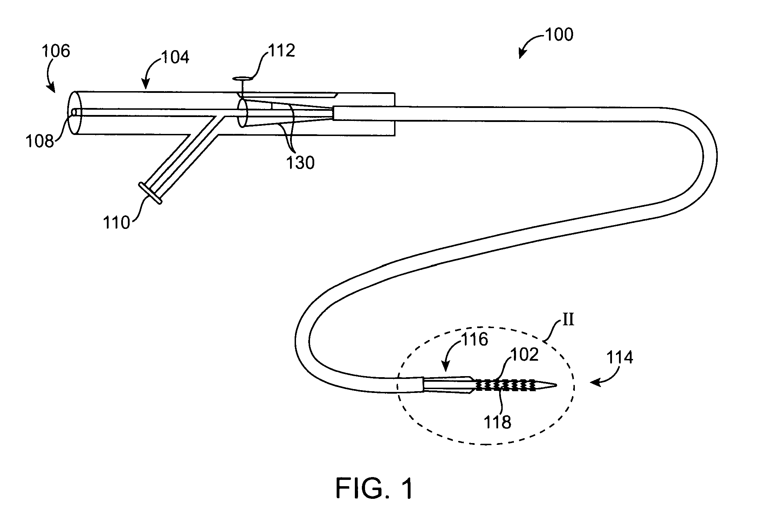

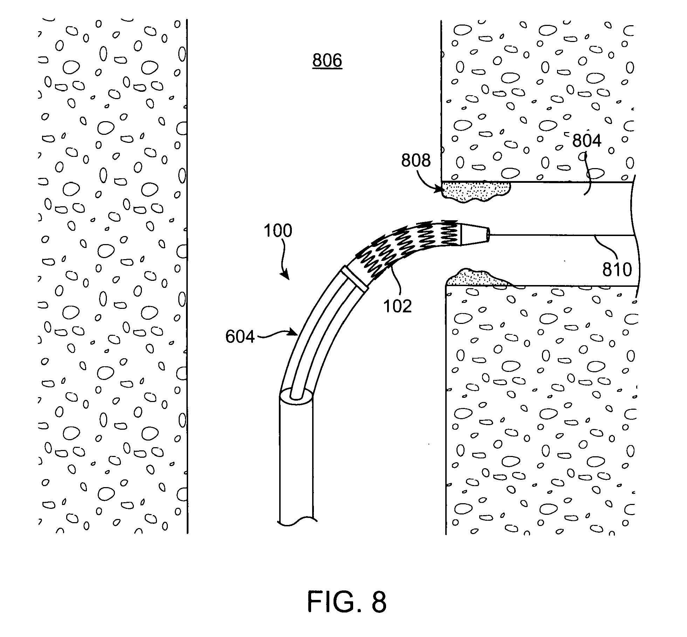

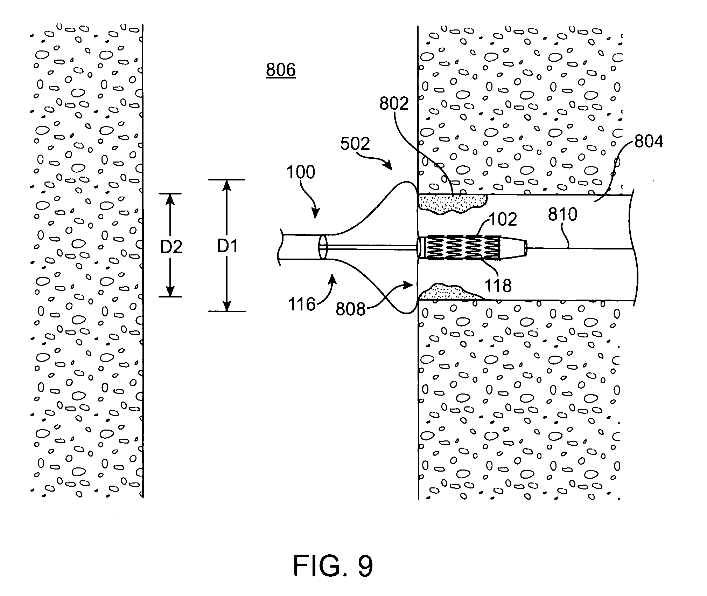

In accordance with one embodiment, referring to FIGS. 8 and 9 together, a method of using a delivery system 100 includes advancing a stent 102 to be located within an ostium 808 of a branch vessel 804; advancing a basket actuation button of a handle (see basket actuation button 112 of handle 104 of FIG. 1 for example) to deploy a basket 502 (FIG. 9) of the basket assembly 116; moving basket 502 into contact (engagement) with a wall of a parent vessel 806, the basket having a larger expanded diameter D1 than a diameter D2 of ostium 808 of branch vessel 804; deploying stent 102 within an ostial lesion 802 of branch vessel 804; and contracting basket 502.

The physician using delivery system 100 feels the force of the expanded basket 502 against the wall of parent vessel 806 simultaneously positioning the stent 102 for precise placement. Such accurate placement can take place with minimal, if any, use of a contrast injection.

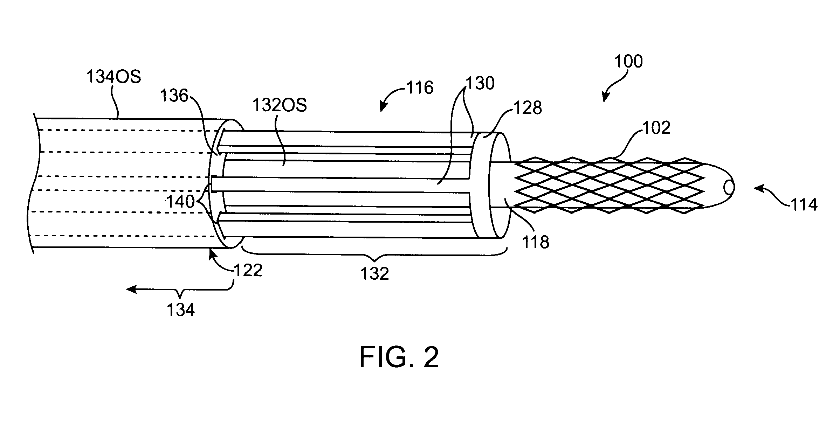

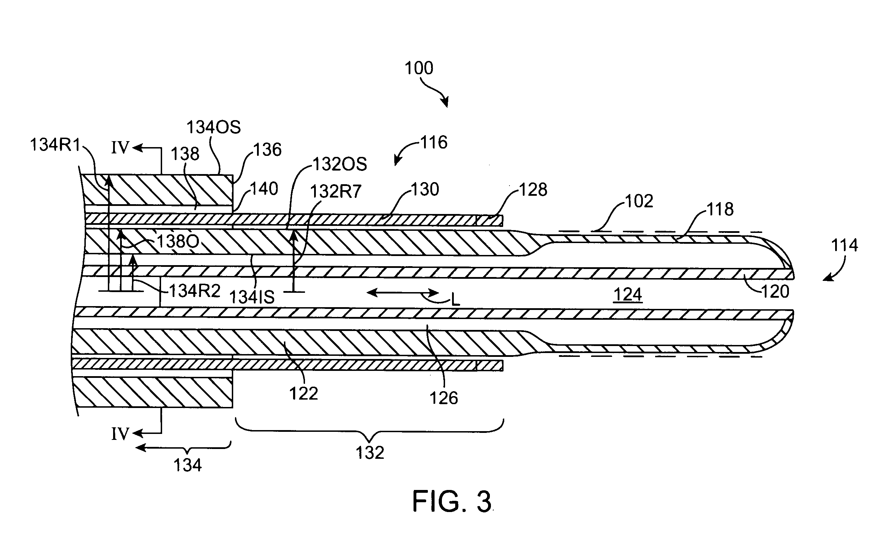

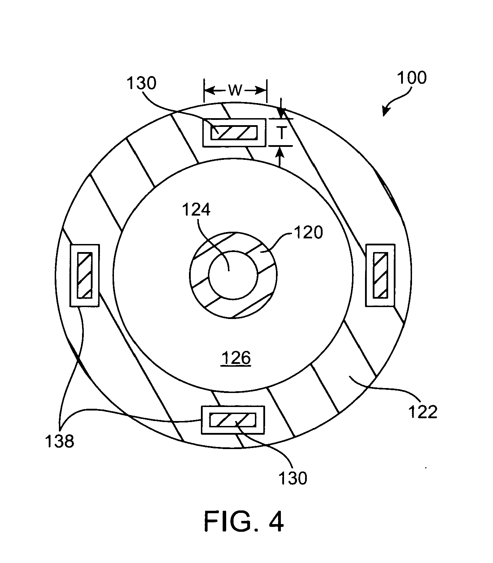

FIG. 1 is a perspective view of a basket delivery system 100 for placement of a stent 102 in an ostial lesion in accordance with one embodiment of the present invention. FIG. 2 is an enlarged perspective view of the region II of basket delivery system 100 of FIG. 1. FIG. 3 is an enlarged cross-sectional view of the region II of basket delivery system 100 of FIG. 1. FIG. 4 is an enlarged cross-sectional view of basket delivery system 100 along the line IV-IV of FIG. 3.

Referring now to FIGS. 1, 2, 3 and 4 together, basket delivery system 100 includes a handle 104 at a proximal end 106 of basket delivery system 100. As used herein, proximal end 106 of basket delivery system 100 is referenced with respect to the operator's handle, i.e., handle 104, while the proximal end of stent 102 is referenced with respect to the end closest to the heart via the length of blood traveled from the heart.

Handle 104 includes a guide wire port 108, a balloon inflation port 110, and a basket actuation button 112, sometimes called a slider.

At a distal end 114 of basket delivery system 100, basket delivery system 100 includes stent 102, a basket assembly 116, and a stent dilation balloon 118. Basket delivery system 100 further includes an inner member 120, and a balloon catheter 122, sometimes called a balloon inflation sheath, extending from handle 104 to distal end 114 of basket delivery system 100.

Inner member 120 is a hollow tube like structure and defines a guide wire lumen 124. As discussed in greater detail below with reference to FIGS. 8 and 9, in one example, basket delivery system 100 is advanced over a guide wire extending through guide wire lumen 124 and out guide wire port 108 of handle 104.

Balloon catheter 122 is also a hollow tube like structure and defines a balloon inflation lumen 126 between balloon catheter 122 and inner member 120. Balloon inflation lumen 126 is communicatively coupled to stent dilation balloon 118 and balloon inflation port 110. As discussed in greater detail below with reference to FIGS. 8 and 9, in one example, stent dilation balloon 118 is inflated and deflated from balloon inflation port 110 and through balloon inflation lumen 126. For example, saline solution is injected into balloon inflation port 110 to inflate stent dilation balloon 118.

Stent 102 is located on stent dilation balloon 118. In accordance with this example, stent 102 is expanded and deploying as discussed in greater detail below with reference to FIGS. 8 and 9 by expansion of stent dilation balloon 118.

Basket assembly 116 is proximally adjacent to stent 102 and stent dilation balloon 118, i.e., basket assembly 116 is proximal and adjacent to stent 102 and stent dilation balloon 118.

Basket assembly 116 includes a basket anchor 128. Basket anchor 128 is fixed to balloon catheter 122 proximally adjacent to stent 102 and stent dilation balloon 118. Accordingly, basket anchor 128 does not move relative to balloon catheter 122.

Basket assembly 116 further includes a least one basket spline 130 connected to basket anchor 128. In this example, basket splines 130 are thin flexible strips, e.g., super-elastic self-expanding strips made of a memory metal such as nitinol although other materials such as polymer can be used. Basket splines 130 extend longitudinally, sometimes called linearly, away from basket anchor 128 in the proximal direction, i.e., towards handle 104.

Balloon catheter 122 includes a basket deployment region 132, sometimes called a first region, proximally adjacent to stent 102 and stent dilation balloon 118. Further, balloon catheter 122 includes a basket pushrod region 134, sometimes called a second region, extending between basket deployment region 132 and handle 104.

In this example, basket deployment region 132 of balloon catheter 122 includes a cylindrical outer surface 132OS having radius 132R1, sometimes called a first radius. Further, basket pushrod region 134 of balloon catheter 122 includes a cylindrical outer surface 134OS having a radius 134R1, sometimes called a second radius. Radius 132R1 of basket deployment region 132 is less than radius 134R1 of basket pushrod region 134.

At the junction of basket deployment region 132 and basket pushrod region 134, balloon catheter 122 further includes a radial annular surface 136. Radial annular surface 136 extends radially outwards from cylindrical outer surface 132OS of basket deployment region 132 to cylindrical outer surface 134OS of basket pushrod region 134.

Basket pushrod region 134 includes a cylindrical inner surface 134IS having a radius 134R2 less than radius 134R1 of outer surface 134OS. Basket pushrod region 134 further includes at least one basket pushrod lumen 138 extending longitudinally in the proximal direction from radial annular surface 136 and between inner surface 134IS and outer surface 134OS of basket pushrod region 134 of balloon catheter 122.

More particularly, basket pushrod lumens 138 are located at a distance 138D from the longitudinal axis L of cylindrical inner surface 134IS and cylindrical outer surface 1340S, cylindrical inner surface 134IS being concentric with cylindrical outer surface 134OS. Distance 138D of basket pushrod lumens 138 is greater than radius 134R2 of cylindrical inner surface 134IS and is less than radius 134R1 of cylindrical outer surface 134OS. Basket pushrod lumens 138 end distally at basket pushrod lumen apertures 140 in radial annular surface 136.

Basket splines 130 extend proximally from basket anchor 128 outside of balloon catheter 122 and adjacent cylindrical outer surface 132OS of basket deployment region 132 of balloon catheter 122. Basket splines 130 pass through basket pushrod lumen apertures 140 and enter basket pushrod lumens 138 within balloon catheter 122. Basket splines 130 extend through basket pushrod lumens 138 all the way to handle 104.

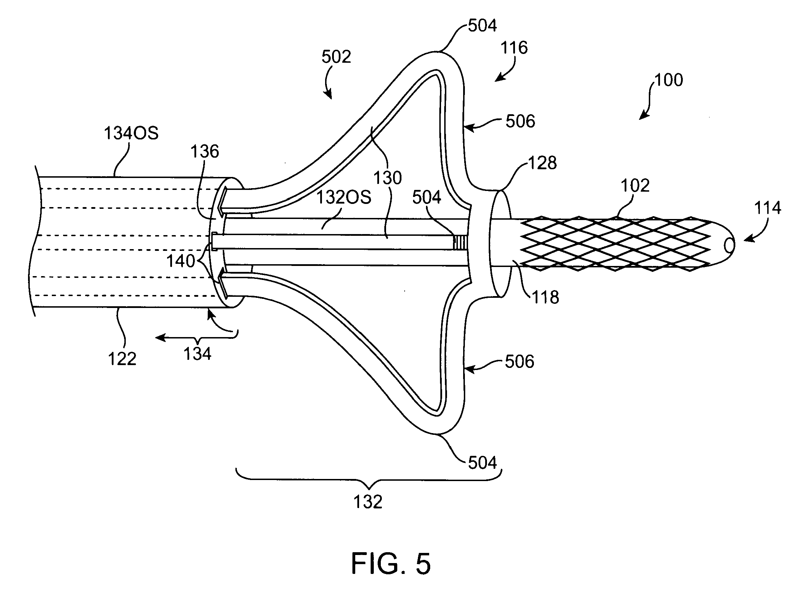

Within handle 104, basket splines 130 are coupled to basket actuation button 112. During use, basket actuation button 112 is advanced, i.e., move distally towards basket assembly 116. Since basket splines 130 are coupled to basket actuation button 112, advancement of basket actuation button 112 pushes basket splines 130 distally through basket pushrod lumens 138. This causes basket splines 130 to feed out of basket pushrod lumen apertures 140 in radial annular surface 136 creating a basket out of basket splines 130 as illustrated FIG. 5.

FIG. 5 is an enlarged perspective view of the region II of basket delivery system 100 of FIG. 1 with basket assembly 116 in its deploying state. Referring now to FIG. 5, as basket splines 130 feed out of basket pushrod lumen apertures 140 in radial annular surface 136, basket splines 130 are forced radially outwards between basket anchor 128 and radial annular surface 136 to define a basket 502, sometimes called an ostial stop or nitinol stop.

Basket splines 130 are rectangular in cross-section (see FIG. 4) having a greater width W than thickness T. Further, in one example, basket splines 130 are super-elastic self-expanding splines set to return to the shape of basket 502. In this manner, basket splines 130 deform preferentially radially outwards instead of randomly such as inwards or sideways.

As shown in FIG. 5, basket 502 is formed of bent basket splines 130. More particularly, basket splines 130 curve radially outwards from outer surface 132OS of basket deployment region 132 to apexes 504 of basket splines 130. In accordance with this example, an apex 504 of a basket spline 130 is the particular location on a basket spline 130 furthest away from outer surface 132OS of basket deployment region 132. Apexes 504 are located just proximal of basket anchor 128 and distally of radial annular surface 136.

As shown in FIG. 5, basket splines 130 extend perpendicularly from basket anchor 128 to apexes 504 to define a forward stop 506. Forward stop 506 generally is in the shape of an annulus to facilitate even contact between forward stop 506 and the wall of the parent vessel.

To collapse basket 502, basket actuation button 112 is retracted, i.e., move proximally away from basket assembly 116. Since basket splines 130 are coupled to basket actuation button 112, retraction of basket actuation button 112 pulls basket splines 130 proximally into basket pushrod lumen apertures 140 in radial annular surface 136 collapsing basket 502.

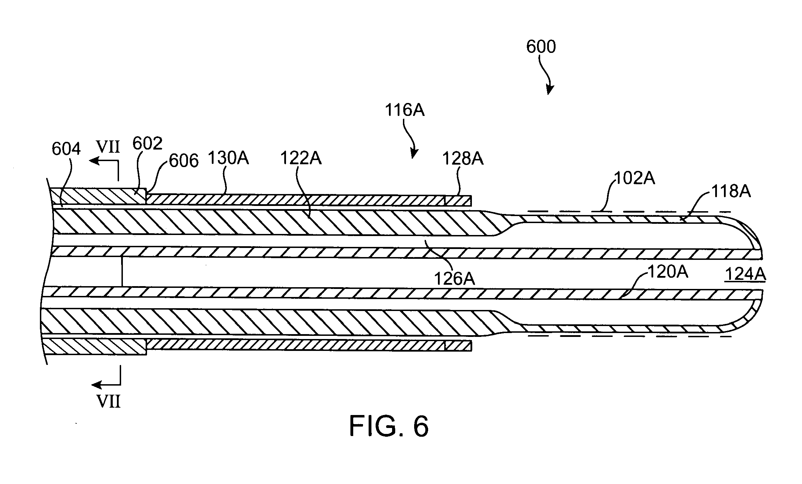

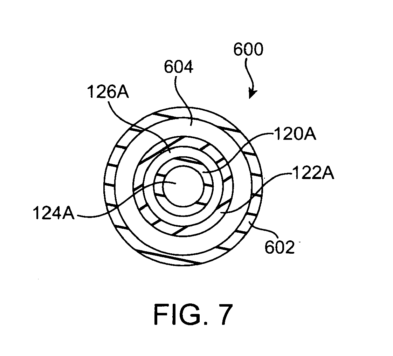

FIG. 6 is an enlarged cross-sectional view of a region of a basket delivery system 600 in accordance with another embodiment. FIG. 7 is a cross-sectional view of basket delivery system 600 along the line VII-VII of FIG. 6.

Basket delivery system 600 of FIGS. 6 and 7 is similar to basket delivery system 100 of FIGS. 1-5 and only the significant differences between basket delivery systems 600 and 100 are discussed below. For example, basket delivery system 600 includes a handle (not shown), a stent 102A, a stent dilation balloon 118A, an inner member 120A, a balloon catheter 122A, a guide wire lumen 124A, and a balloon inflation lumen 126A which are similar to handle 104, stent 102, stent dilation balloon 118, inner member 120, balloon catheter 122, guide wire lumen 124, and balloon inflation lumen 126 of basket delivery system 100.

Referring now to FIGS. 6 and 7 together, basket delivery system 600 includes a basket assembly 116A. Basket assembly 116A is proximally adjacent to stent 102A and stent dilation balloon 118A. Basket assembly 116A includes a basket anchor 128A. Basket anchor 128A is fixed to balloon catheter 122A proximally adjacent to stent 102A and stent dilation balloon 118A. Accordingly, basket anchor 128A does not move relative to balloon catheter 122A.

Basket assembly 116A further includes a least one basket spline 130A connected to basket anchor 128A. In this example, basket splines 130A are thin flexible strips, e.g., made of a memory metal such as nitinol. Basket splines 130A extend longitudinally away from basket anchor 128A in the proximal direction.

In this example, balloon catheter 122A is a hollow tube-like member. Delivery system 600 further includes a basket deployment sheath 602. Basket deployment sheath 602 is also a hollow tube-like member and includes a balloon catheter lumen 604. Balloon catheter 122A is located within balloon catheter lumen 604.

Basket deployment sheath 602 is connected to basket splines 130A at a distal end 606 of basket deployment sheath 602 and to a basket actuation button of a handle (similar to basket actuation button 112 of handle 104 of basket delivery system 100 of FIG. 1) at a proximal end of basket deployment sheath 602.

To deploy the basket of basket assembly 116A, the basket actuation button of the handle is advanced. Since basket deployment sheath 602 is coupled to the basket actuation button, advancement of the basket actuation button pushes basket deployment sheath 602 distally over balloon catheter 122A. This causes basket splines 130A to be compressed between distal end 606 of basket deployment sheath 602 and basket anchor 128A creating a basket out of basket splines 130A similar to basket 502 as illustrated in FIG. 5.

To collapse the basket, the basket actuation button is retracted. Since basket deployment sheath 602 is coupled to the basket actuation button, retraction of the basket actuation button pulls basket deployment sheath 602 proximally over balloon catheter 122A collapsing the basket.

FIG. 8 is a sectional schematic perspective view of the advancement of basket delivery system 100 of FIGS. 1-5 to an ostial lesion 802 of an artery 804 in accordance with one embodiment of the present invention. Referring now to FIG. 8, artery 804, e.g., a renal artery, sometimes called a branch vessel, is branching from a parent vessel 806, e.g., the aorta, and includes an ostium 808. Ostial lesion 802 is located within artery 804 adjacent ostium 808.

Basket delivery system 100 is advanced over a guide wire 810. In one example, basket delivery system 100 is advanced through a delivery catheter (not shown).

Basket delivery system 100 is advanced until stent 102 is located within ostium 808 and basket assembly 116 is located within parent vessel 806 directly adjacent ostium 808.

FIG. 9 is a sectional schematic perspective view of the further advancement of basket delivery system 100 of FIG. 8 to ostial lesion 802 of artery 804. Referring now to FIGS. 8 and 9 together, basket 502 is deployed as discussed above in reference to FIG. 5. The outer diameter of the apexes of the expanded basket 502 has a diameter D1 larger than a diameter D2 of ostium 808. Basket delivery system 100 is advanced until basket 502 stops against the wall of parent vessel 806 as shown in FIG. 9. The physician using basket delivery system 100 feels the engagement (contact) of basket 502 against the wall of parent vessel 806 confirming the correct placement of the catheter carrying stent 102. Stent 102 is positioned accurately using basket delivery system 100 without the use of contrast in one example. However, if desired, the physician can use a small amount of contrast to further confirm the correct placement of stent 102.

More particularly, once basket 502 is securely engaged (in contact with) against the wall of parent vessel 806, stent 102 is positioned within ostial lesion 802. While the physician maintains forward pressure on basket delivery system 100, stent dilation balloon 118 is expanded thus expanding and deploying stent 102 within ostial lesion 802. Stent dilation balloon 118 is deflated, basket 502 is contracted, and basket delivery system 100 is withdrawn from the patient.

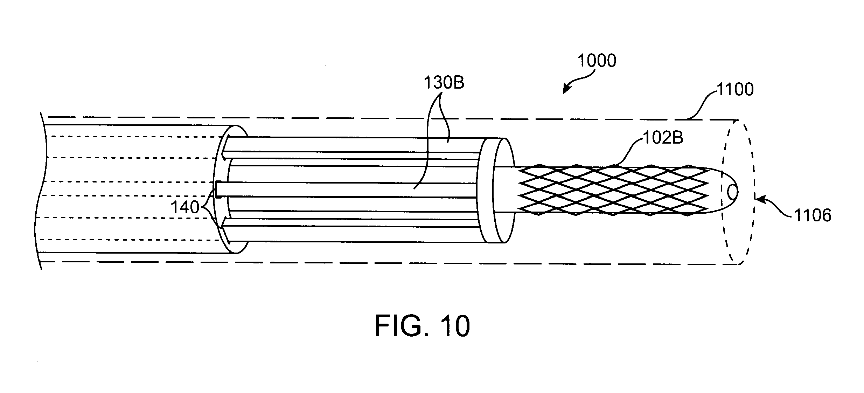

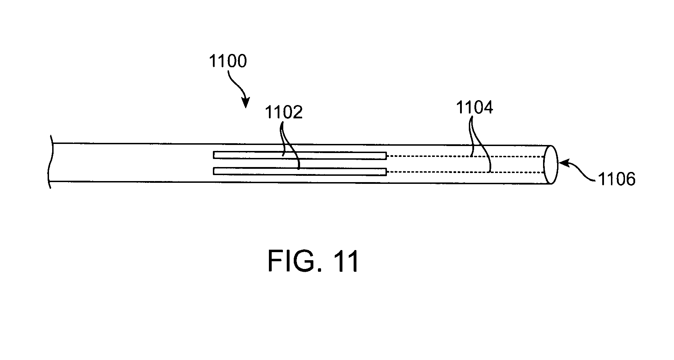

FIG. 10 is a schematic perspective view of a basket delivery system 1000 in accordance with another embodiment. FIG. 11 is a schematic perspective view of a stent delivery sheath 1100 of basket delivery system 1000 of FIG. 10. In FIG. 10, stent delivery sheath 1100 is illustrated by dashed lines. Basket delivery system 1000 of FIG. 10 is similar to basket delivery system 100 of FIG. 1 and only the significant differences between basket delivery systems 1000 and 100 are discussed below.

Referring now to FIGS. 10 and 11 together, basket delivery system 1000 includes a self-expanding stent 102B. Self-expanding stent 102B is constrained within stent delivery sheath 1100. Upon retraction of stent delivery sheath 1100 and exposure of self-expanding stent 102B, self-expanding stent 102B self expands. Accordingly, since self-expanding stent 102B self expands, in one example, basket delivery system 1000 does not include a stent dilation balloon.

Stent delivery sheath 1100 includes at least one longitudinal slot 1102 corresponding to basket splines 130B. In accordance with this example, stent delivery sheath 1100 further includes at least one breakaway 1104 corresponding to slots 1102. Generally, a breakaway 1104 is a region of stent delivery sheath 1100 having a reduced strength such that the breakaway 1104 readily tears upon application of a force to the breakaway. Illustratively, breakaways 1104 are scores or perforations in stent delivery sheath 1100.

More particularly, stent delivery sheath 1100 includes a slot 1102 for each basket spline 130B. Slots 1102 are aligned with basket splines 130B by fixing the placement of stent delivery sheath 1100 relative to basket splines 130B, for example, by a tab or other structure of stent delivery sheath 1100 that allows relative longitudinal but not rotational motion. Breakaways 1104 extend distally from slots 1102 to a distal end 1106 of stent delivery sheath 1100.

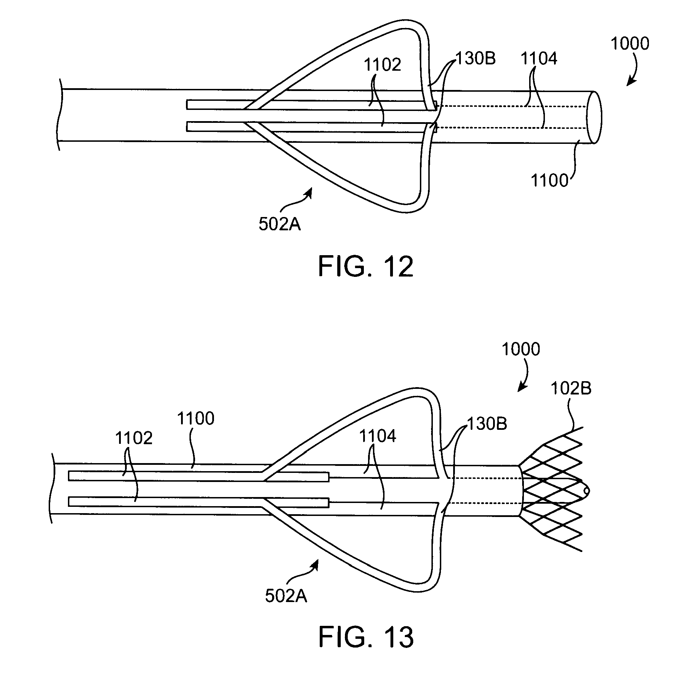

FIG. 12 is a perspective view of basket delivery system 1000 of FIG. 10 having a basket 502A deployed. As shown in FIG. 12, basket splines 130B are bent radially outwards through slots 1102 to deploy basket 502A in a manner similar to that discussed with regards to deployment of basket 502 of basket delivery system 100 of FIG. 5.

FIG. 13 is a perspective view of basket delivery system 1000 of FIG. 12 during deployment of self-expanding stent 102B. Referring now to FIG. 13, stent delivery sheath 1100 is retracted, for example, through retraction of an actuation button of a handle, the actuation button being coupled to delivery sheath 1100. Retraction of stent delivery sheath 1100 causes stent delivery sheath 1100 to move relative to basket 502A. Basket 502A, and more particularly, basket splines 130B are forced against breakaways 1104. This causes breakaways 1104 to begin to tear.

Further, retraction of stent delivery sheath 1100 causes breakaways 1104 to continue to tear and exposes self-expanding stent 102B. Upon exposure, self-expanding stent 102B self expands, for example, into an ostial lesion.

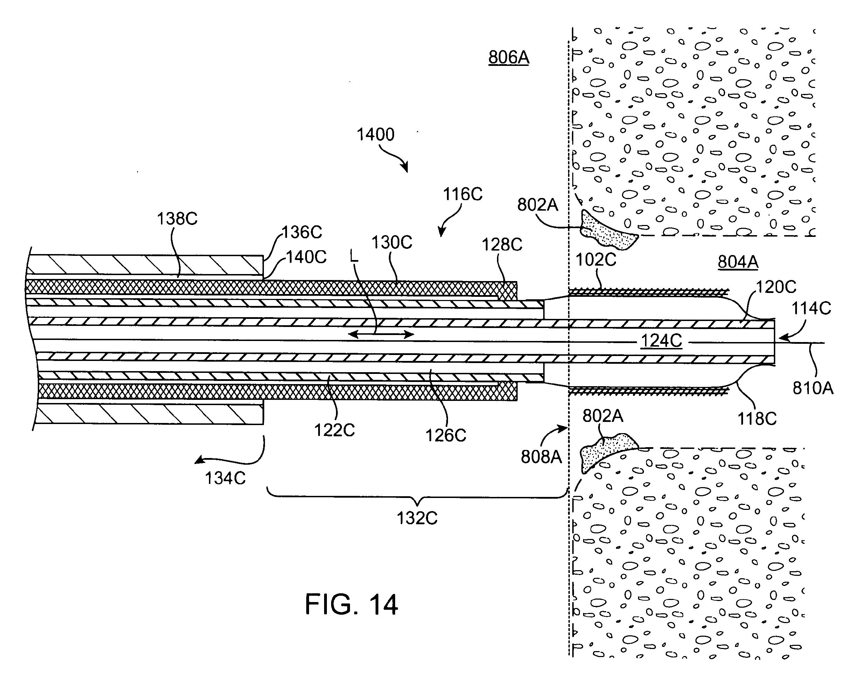

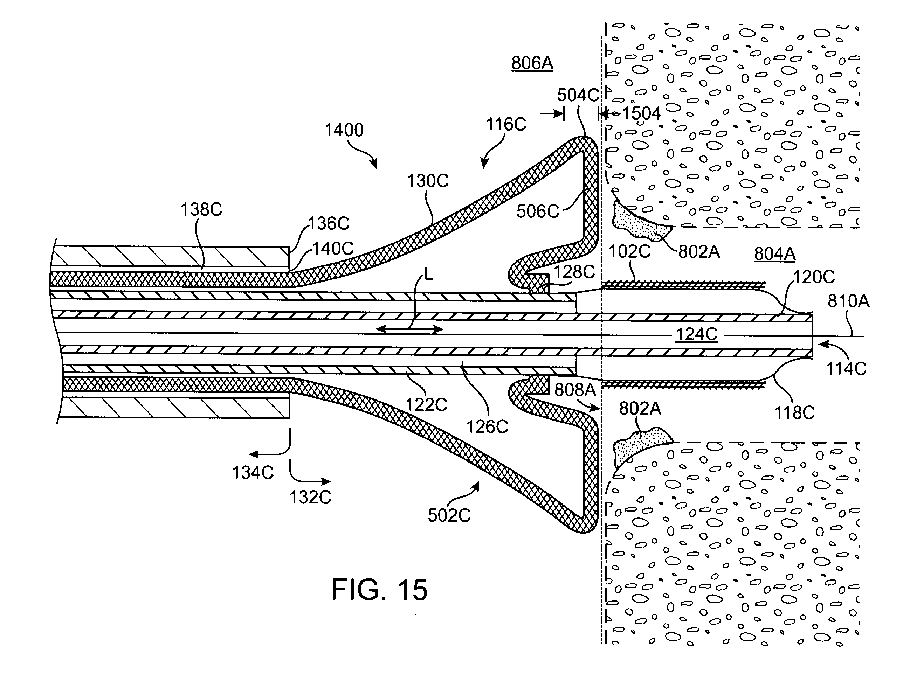

FIG. 14 is an enlarged cross-sectional view of a region of a basket delivery system 1400 during advancement to an ostial lesion 802A of an artery 804A in accordance with another embodiment. FIG. 15 is an enlarged cross-sectional view of the region of basket delivery system 1400 during advancement to ostial lesion 802A of artery 804A of FIG. 14 with a basket assembly 116C in its deploying state.

Referring now to FIGS. 3, 14, and 15 together, basket delivery system 1400 of FIGS. 14, 15 is similar to basket delivery system 100 of FIG. 3 and only the significant differences are discussed below. More particularly, basket delivery system 1400 of FIGS. 14, 15 includes a stent 102C, a distal end 114C, a stent dilation balloon 118C, an inner member 120C, a balloon catheter 122C, a guide wire lumen 124C, a balloon inflation lumen 126C, a basket anchor 128C, basket splines 130C, a basket deployment region 132C, a basket pushrod region 134C, a radial annular surface 136C, basket pushrod lumens 138C and basket pushrod lumen apertures 140C similar to stent 102, distal end 114, stent dilation balloon 118, inner member 120, balloon catheter 122, guide wire lumen 124, balloon inflation lumen 126, basket anchor 128, basket splines 130, basket deployment region 132, basket pushrod region 134, radial annular surface 136, basket pushrod lumens 138 and basket pushrod lumen apertures 140 of basket delivery system 100 of FIG. 3, respectively.

Referring now to FIG. 15, after advancement of basket delivery system 1400 over guidewire 810A, basket splines 130C are fed out of basket pushrod lumen apertures 140C in radial annular surface 136C. As a result, basket splines 130C are forced radially outwards between basket anchor 128C and radial annular surface 136C to define a basket 502C, sometimes called an ostial stop or nitinol stop.

In one example, basket splines 130C are super-elastic self-expanding splines set to return to the shape of basket 502C. In this manner, basket splines 130C deform preferentially radially outwards instead of randomly such as inwards or sideways.

As shown in FIG. 15, basket 502C is formed of bent basket splines 130C. More particularly, basket splines 130C curve radially outwards from radial annular surface 136C to apexes 504C of basket splines 130C. Apexes 504C are located just distal to basket anchor 128C and proximal to stent 102C.

As shown in FIG. 15, basket splines 130C extend distally from and over basket anchor 128C to apexes 504C to define a forward stop 506C. Forward stop 506C generally is in the shape of an annulus to facilitate even contact between forward stop 506C and the wall of parent vessel 806A.

Forward stop 506C is distal of basket anchor 128C and proximal of stent 102C in this example. By defining the forward stop distance 1504 that basket splines 130C extend distally past basket anchor 128C, the placement of stent 102C within ostium 808A is also defined.

Stated another way, forward stop 506C is distal to basket anchor 128C by the forward stop distance 1504. As set forth above, forward stop 506C contacts the wall of parent vessel 806A and thus defines the position of basket delivery system 1400 and stent 102C with respect to the wall of parent vessel 806A. By defining the forward stop distance 1504, the position of stent 102C with respect to the wall of parent vessel 806A is also defined. Illustratively, basket delivery systems similar to basket delivery system 1400 are formed with greater or less forward stop distances than forward stop distance 1504 to assure that the stent deployed by an expanding balloon which extends beyond to the ends of the stent on the catheter is fully deployed and positions the stent edge right at the ostium.

To collapse basket 502C, the basket actuation button is retracted, i.e., move proximally away from basket assembly 116C. Since basket splines 130C are coupled to the basket actuation button, retraction of the basket actuation button pulls basket splines 130C proximally into basket pushrod lumen apertures 140C in radial annular surface 136C collapsing basket 502C.

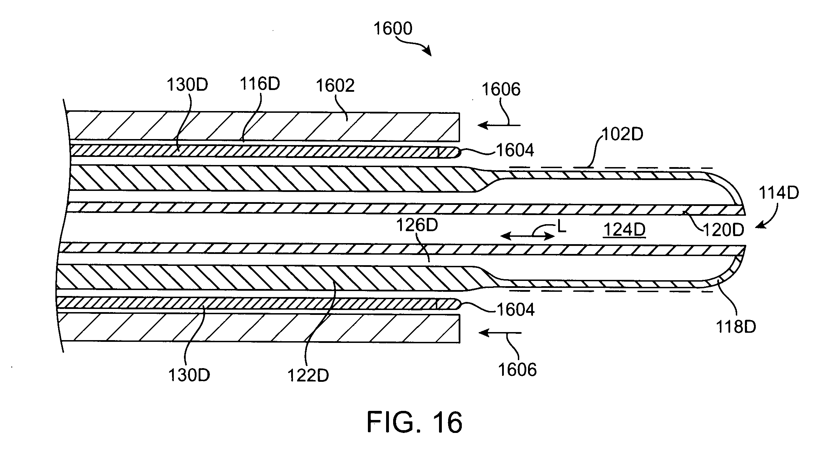

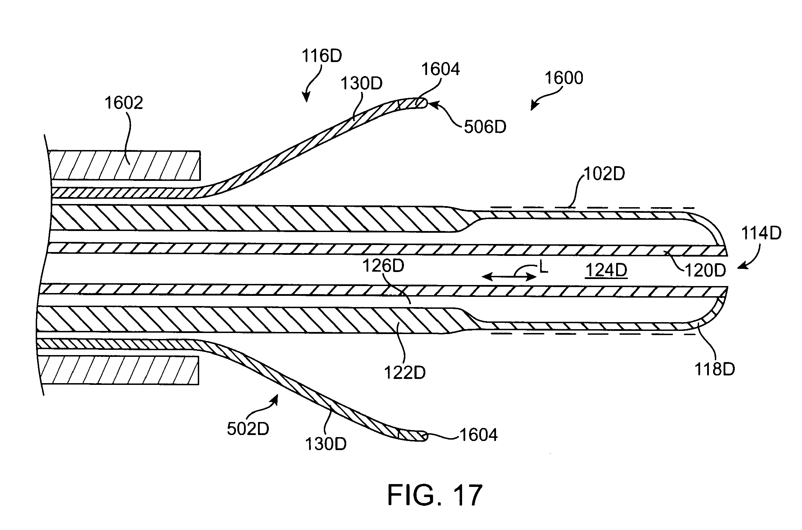

FIG. 16 is an enlarged cross-sectional view of a region of a basket delivery system 1600 in accordance with another embodiment. FIG. 17 is an enlarged cross-sectional view of the region of basket delivery system 1600 of FIG. 16 with a basket assembly 116D in its deploying state.

Referring now to FIGS. 3, 16, and 17 together, basket delivery system 1600 of FIGS. 16, 17 is similar to basket delivery system 100 of FIG. 3 and only the significant differences are discussed below. More particularly, basket delivery system 1600 of FIGS. 16, 17 includes a stent 102D, a distal end 114D, a stent dilation balloon 118D, an inner member 120D, a balloon catheter 122D, a guide wire lumen 124D, a balloon inflation lumen 126D, and basket splines 130D similar to stent 102, distal end 114, stent dilation balloon 118, inner member 120, balloon catheter 122, guide wire lumen 124, balloon inflation lumen 126, and basket splines 130 of basket delivery system 100 of FIG. 3, respectively.

Referring now to FIG. 16, basket delivery system 1600 includes a sheath 1602 over basket splines 130D. In accordance with this example, basket splines 130D are super-elastic self-expanding splines constrained within sheath 1602. Basket splines 130D terminate at the distal end of basket splines 130D at basket spline tips 1604. In various examples, basket spline tips 1604 are made of nitinol, stainless steel, plastic, or other material.

To deploy basket splines 130D, sheath 1602 is retracted proximally and in the direction of arrows 1606. For example, a handle similar to handle 104 of FIG. 1 includes a sheath actuation button coupled to sheath 1602. The sheath actuation button is retracted and advanced to retract and advance sheath 1602.

As sheath 1602 is retracted, basket splines 130D are uncovered, sometimes called exposed. As basket splines 130D are exposed, basket splines 130D self-expand. This self-expansion of basket splines 130D causes basket spline tips 1604 to spread apart from one another and generally to become spaced from balloon catheter 122D as shown in FIG. 17. Accordingly, basket splines 130D form a basket 502D including a forward stop 506D defined by basket spline tips 1604.

To collapse basket 502D, sheath 1602 is advanced distally and in the direction opposite of arrows 1606. As sheath 1602 is advanced, sheath 1602 contacts and collapses basket splines 130D as sheath 1602 passes over basket splines 130D. Sheath 1602 is advanced until basket splines 130D are again located within and constrained by sheath 1602 as shown in FIG. 16.

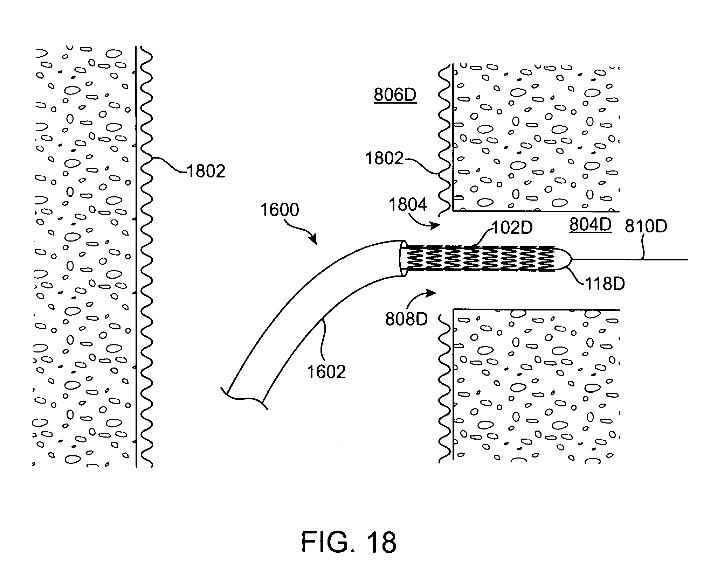

FIGS. 18, 19, 20 are sectional schematic perspective views of the advancement and deployment of basket delivery system 1600 of FIGS. 16, 17. Referring now to FIG. 18, artery 804D, e.g., a renal artery, sometimes called a branch vessel, is branching from a parent vessel 806D, e.g., the aorta, and includes an ostium 808D. A graft 1802 is deployed within parent vessel 806D using any one of a number of techniques well known to those of skill in the art.

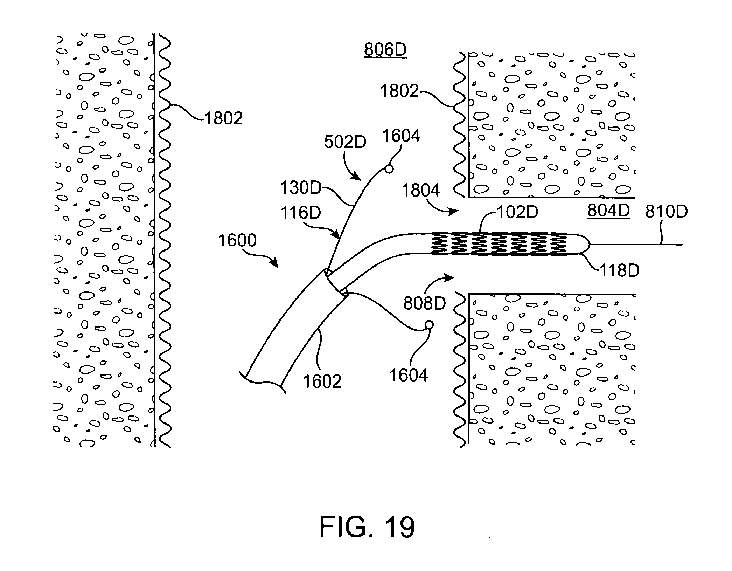

Graft 1802 includes a branch vessel opening 1804 aligned with artery 804D. Basket delivery system 1600 is advanced over a guide wire 810D and through branch vessel opening 1804. Basket delivery system 1600 is advanced until stent 102D, sometimes called a branch stent, is located within or near ostium 808D and/or branch vessel opening 1804. Basket assembly 116D (covered by sheath 1602 in the view of FIG. 18, see FIG. 19) is located within parent vessel 806D and graft 1802 directly adjacent ostium 808D and branch vessel opening 1804.

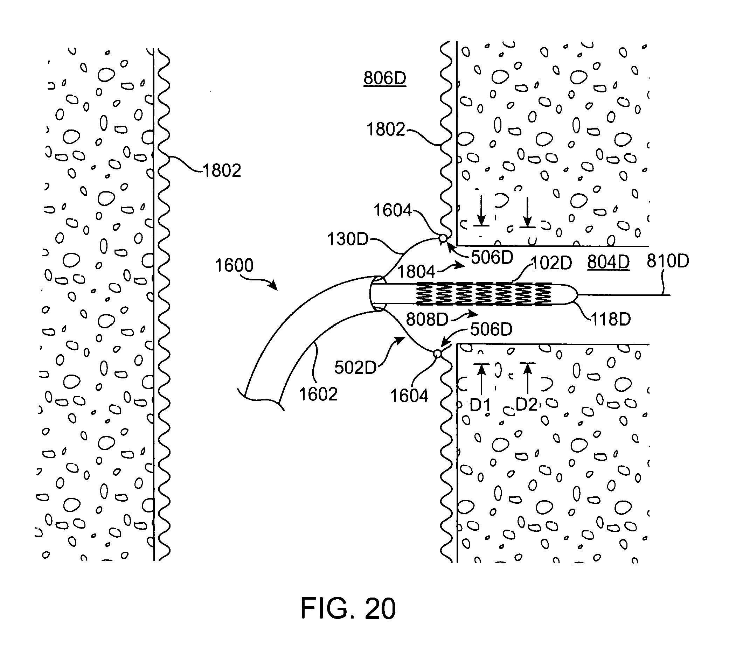

Referring now to FIG. 19, sheath 1602 is retracted thus deploying basket splines 130D as discussed above. Referring now to FIG. 20, basket delivery system 1600 is advanced, sometimes called moved, until basket spline tips 1604 press against graft 1802 and the wall of parent vessel 806D.

As shown in FIG. 20, the outer diameter of forward stop 506D defined by basket spline tips 1604 of the expanded basket 502D has a diameter D1 larger than a diameter D2 of branch vessel opening 1804. Basket delivery system 1600 is advanced until basket 502D stops against the wall of graft 1802 as shown in FIG. 20. The physician using basket delivery system 1600 feels the engagement (contact) of basket 502D against the wall of graft 1802 confirming the correct placement of the catheter carrying stent 102D. Stent 102D is positioned accurately using basket delivery system 1600 without the use of contrast in one example. However, if desired, the physician can use a small amount of contrast to further confirm the correct placement of stent 102D.

More particularly, once basket 502D is securely engaged (in contact with) against the wall of graft 1802, stent 102D is positioned within ostium 808D and/or branch vessel opening 1804. While the physician maintains forward pressure on basket delivery system 1600, stent dilation balloon 118D is expanded thus expanding and deploying stent 102D within ostium 808D and/or branch vessel opening 1804. Stent dilation balloon 118D is deflated, basket 502D including basket splines 130D is collapsed by advancement of sheath 1602 as discussed above, and basket delivery system 1600 is withdrawn from the patient.

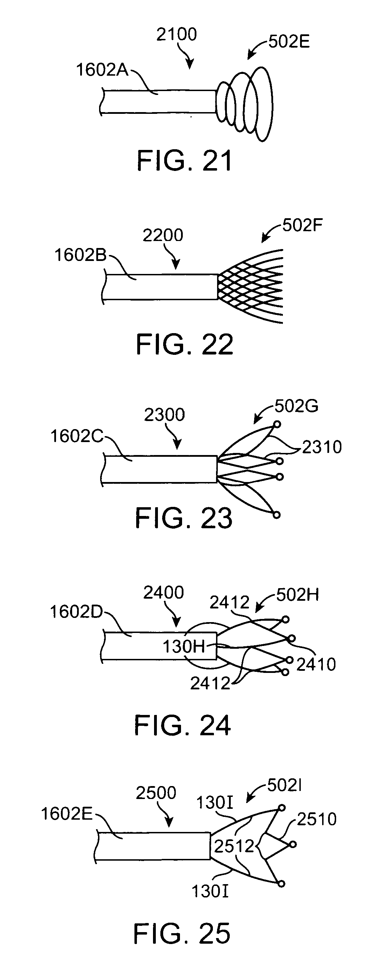

Although basket delivery system 1600 includes basket 502D formed of basket splines 130D, in other examples, a basket delivery system includes a basket having other shapes. For example, FIG. 21 is a perspective view of a region of a basket delivery system 2100 having a basket 502E in the form of a spiral wire in accordance with another embodiment. Generally, basket 502E is a single spiral having a minimum diameter at a sheath 1602A and radially increasing in diameter distally from sheath 1602A.

For purposes of simplicity, only a sheath 1602A and a basket 502E of basket delivery system 2100 is illustrated in FIG. 21. However, apart from basket 502E, it is to be understood that basket delivery system 2100 of FIG. 21 is substantially similar to basket delivery system 1600 of FIG. 16 and includes similar elements.

For another example, FIG. 22 is a perspective view of a region of a basket delivery system 2200 having a basket 502F in the form of braided wires in accordance with another embodiment. Generally, basket 502F is formed of braided wires, e.g., a mesh. Basket 502F has a minimum diameter at a sheath 1602B and radially increases in diameter distally from sheath 1602B.

For purposes of simplicity, only a sheath 1602B and a basket 502F of basket delivery system 2200 is illustrated in FIG. 22. However, apart from basket 502F, it is to be understood that basket delivery system 2200 of FIG. 22 is substantially similar to basket delivery system 1600 of FIG. 16 and includes similar elements.

For another example, FIG. 23 is a perspective view of a region of a basket delivery system 2300 having a basket 502G in the form of diamond shaped segments 2310 in accordance with another embodiment. Basket 502G has a minimum diameter at a sheath 1602C and radially increases in diameter distally from sheath 1602C.

For purposes of simplicity, only a sheath 1602C and a basket 502G of basket delivery system 2300 is illustrated in FIG. 23. However, apart from basket 502G, it is to be understood that basket delivery system 2300 of FIG. 23 is substantially similar to basket delivery system 1600 of FIG. 16 and includes similar elements.

For another example, FIG. 24 is a perspective view of a region of a basket delivery system 2400 having a basket 502H in accordance with another embodiment. Generally, basket 502H is in the form of a distal ring segment 2410 having an alternating repeating pattern. Each proximal crown 2412 of distal ring segment 2410 is connected to a spline 130H. Basket 502H has a minimum diameter at sheath 1602D and radially increases in diameter distally from sheath 1602D. [00100] For purposes of simplicity, only a sheath 1602D and a basket 502H of basket delivery system 2400 is illustrated in FIG. 24. However, apart from basket 502H, it is to be understood that basket delivery system 2400 of FIG. 24 is substantially similar to basket delivery system 1600 of FIG. 16 and includes similar elements.

For another example, FIG. 25 is a perspective view of a region of a basket delivery system 2500 having a basket 502I in accordance with another embodiment. Generally, basket 502I is formed of distal ring segment 2510 having an alternating repeating pattern. Some of proximal crowns 2512 of distal ring segment 2510 are connected to splines 1301 and some of proximal crowns 2512 of distal ring segment 2510 are unconnected. In one example, the unconnected proximal crowns 2512 are bent radially inwards to prevent the unconnected proximal crowns 2512 from catching on sheath 1602E during advancement and recapture of basket 502I. Basket 502I has a minimum diameter at sheath 1602E and radially increases in diameter distally from sheath 1602E.

For purposes of simplicity, only a sheath 1602E and a basket 502I of basket delivery system 2500 is illustrated in FIG. 25. However, apart from basket 502I, it is to be understood that basket delivery system 2500 of FIG. 25 is substantially similar to basket delivery system 1600 of FIG. 16 and includes similar elements.

This disclosure provides exemplary embodiments according to the present invention. Numerous variations, whether explicitly provided for by the specification or implied by the specification or not, such as variations in structure, dimension, type of material and manufacturing process may be implemented by one of skill in the art in view of this disclosure.

## Claims

1. A delivery system (100) comprising:
a stent dilation balloon (118) at a distal end (114) of said delivery system (100);
a stent (102) on said stent dilation balloon (118); and
a basket assembly (116) proximally adjacent to said stent dilation balloon (118), said basket assembly (116) comprising at least one super-elastic self-expanding basket spline (130).

2. The delivery system of Claim 1 further comprising a balloon catheter (122), a basket anchor (128) of said basket assembly (116) being coupled to said balloon catheter (122).

3. The delivery system of Claim 2 further comprising an inner member (120), wherein a balloon inflation lumen (126) is defined between said balloon catheter (122) and said inner member (120).

4. The delivery system of Claim 3 wherein said stent dilation balloon (118) is communicatively coupled to said balloon inflation lumen (126).

5. The delivery system of any of Claims 1 to 4 wherein said inner member (120) defines a guide wire lumen (124).

6. The delivery system of any of Claims 1 to 5 wherein said at least one super-elastic self-expanding basket spline (130) comprises a thin flexible strip.

7. The delivery system of Claim 6 wherein said thin flexible strip comprises a memory metal.

8. The delivery system of any of Claims 1 to 7 further comprising a handle (104) and a balloon catheter (122), said balloon catheter (122) comprising:
a basket deployment region (132) proximally adjacent to said stent (102); and
a basket pushrod region (134) extending between said basket deployment region (132) and said handle (104).

9. The delivery system of Claim 8 wherein said basket deployment region (132) comprises a cylindrical outer surface (132OS) having a first radius (132R1) and wherein said basket pushrod region (134) comprises a cylindrical outer surface (1340S) having a second radius (134R1), said first radius (132R1) being less than said second radius (134R1).

10. The delivery system of Claim 8 or 9 wherein said balloon catheter (122) further comprises a radial annular surface (136) at a junction of said basket deployment region (132) and said basket pushrod region (134).

11. The delivery system of Claim 10 wherein said radial annular surface (136) extends radially outwards from said cylindrical outer surface (132OS) of said basket deployment region (132) to said cylindrical outer surface (134OS) of said basket pushrod region (134).

12. The delivery system of any of Claims 8 to 11 wherein said basket pushrod region (134) comprises at least one basket pushrod lumen (138).

13. The delivery system of Claim 12 wherein said at least one basket pushrod lumen (138) ends distally from at least one basket pushrod lumen aperture (140) in said radial annular surface (136).

14. The delivery system of Claim 12 or 13 wherein said at least one super-elastic self-expanding basket spline (130) extends through said at least one basket pushrod lumen (138) and to said handle (104), said handle (104) further comprising a basket actuation button (112) coupled to said at least one super-elastic self-expanding basket spline (130).

15. The delivery system of any of Claims 2 to 14 wherein said at least one super-elastic self-expanding basket spline (130) extends proximally from the basket anchor (138) coupled to said balloon catheter (122) adjacent a cylindrical outer surface (132OS) of a basket deployment region (132) and into at least one basket pushrod lumen (138).

16. The delivery system of any of Claims 1 to 15 wherein said at least one super-elastic self-expanding basket spline (130) is bent to form a basket (502).

17. The delivery system of Claim 16 wherein said at least one super-elastic self-expanding basket spline (130) curves radially outwards to apexes (504).

18. The delivery system of any of Claims 1 to 17 further comprising a basket deployment sheath (602) coupled to said at least one super-elastic self-expanding basket spline (130A) at a distal end (606) of said basket deployment sheath (602).

19. The delivery system of Claim 18 further comprising a handle comprising a basket actuation button, a proximal end of said basket deployment sheath (602) being coupled to said basket actuation button.

20. The delivery system of any of Claims 1 to 19 wherein said basket (502C) comprises a forward stop (506C).

21. The delivery system of Claim 20 further comprising a balloon catheter (122C), wherein a basket anchor (128C) of said basket assembly (116C) is coupled to said balloon catheter (122C), and wherein said forward stop (506C) is distal to said basket anchor (128C).

22. The delivery system of Claim 20 or 21 wherein said forward stop (506C) is in the shape of an annulus.

23. The delivery system of any of Claims 20 to 22 wherein said forward stop (506C) is proximal of said stent (102C).

24. The delivery system of any of Claims 1 to 23 wherein said at least one super-elastic self-expanding basket spline (130D) terminate at basket spline tips (1604).

25. The delivery system of Claim 24 wherein said basket spline tips (1604) comprise a material selected from the group consisting of nitinol, stainless steel and plastic.

26. The delivery system of Claim 24 or 25 further comprising a sheath (1602) constraining said at least one super-elastic self-expanding basket spline (130D).

27. The delivery system of any of Claims 24 to 26 wherein said basket spline tips (1604) define a forward stop.

28. The delivery system according to any of the preceding claims, wherein
said basket is a self-expanding basket proximally adjacent to said stent dilation balloon.

29. The delivery system of any of Claims 1 to 28 wherein said basket (502E) is in the form of a spiral wire.

30. The delivery system of any of Claims 1 to 28 wherein said basket (502F) is in the form of braided wires.

31. The delivery system of any of Claims 1 to 28 wherein said basket (502G) is in the form of diamond shaped segments.

32. The delivery system of any of Claims 1 to 28 wherein said basket (502H) is in the form of a distal ring segment (2410, 2510) having an alternating repeating pattern.

33. The delivery system of Claim 32 wherein each proximal crown (2412) of said distal ring segment (2410) is connected to a spline (130H) of said basket (502H).

34. The delivery system of Claim 32 wherein some proximal crowns (2512) of said distal ring segment (2510) are unconnected.

35. The delivery system of any of the preceding claims, further comprising a sheath (1602E) for deploying and recapturing said basket, wherein said basket has a minimum diameter at said sheath and radially increases in diameter distally from said sheath (1602E).

36. A delivery system (1000) comprising:
a self-expanding stent (102B) at a distal end of said delivery system (1000);
a basket assembly proximally adjacent said stent (102B), said basket assembly comprising at least one super-elastic self-expanding basket spline (130B); and
a stent delivery sheath (1100) constraining said self-expanding stent (102B).

37. The delivery system of Claim 36 wherein said stent delivery sheath (1100) comprises at least one slot (1102) aligned with said at least one super-elastic self-expanding basket spline (130B).

38. The delivery system of Claim 37 wherein said stent delivery sheath (1100) further comprises at least one breakaway (1104) corresponding to said at least one slot (1102).

39. The delivery system of Claim 38 wherein said at least one breakaway (1104) readily tears upon application of force to said at least one breakaway (1104).

40. The delivery system of Claim 38 or 39 wherein said at least one breakaway (1104) comprises scores in said stent delivery sheath (1100).

41. The delivery system of any of Claims 38 to 40 wherein said at least one breakaway (1104) comprises perforations in said stent delivery sheath (1100).
